# EUROPEAN PATENT SPECIFICATION

(11) **EP 3 801 315 B1**
(45) Date of publication and mention of the grant of the patent: **07.02.2024**
(21) Application number: 19815861.0
(22) Date of filing: 09.04.2019
(51) Int. Cl.: A61B 17/34, A61B 10/02, A61M 5/158

(54) **INTRAOSSEOUS INFUSION DEVICE**
VORRICHTUNG ZUR INTRAOSSALEN INFUSION
DISPOSITIF DE PERFUSION INTRA-OSSEUSE

(30) Priority: 07.06.2018 TR 201808113
(43) Date of publication of application: 14.04.2021
(73) Proprietor: Matek Medikal Aygitlar Teknoloji Sanayi Ve Ticaret Anonim Sirketi, Ankara (TR)
(72) Inventor: YAGMURLU, Musa, Ankara (TR)
(74) Representative: Berkkam, Ayfer
(86) International application number: PCT/TR2019/050231
(87) International publication number: WO 2019/236042

(56) References cited:
- US-A1- 2003 225 411
- US-A1- 2003 225 411
- US-A1- 2008 215 056
- US-A1- 2011 082 387
- US-A1- 2014 276 205
- US-A1- 2014 276 205

## Description

### Technical field

In general, the invention is related to a medical device that is used in bone marrow intervention.

More specifically, the invention is related to a disposable device that is used for intraosseous infusion, that comprises a drive comprising a motor and a power unit and a trocar integrated with said driver.

### Prior Art

In general, the intraosseous infusion procedure is defined as inserting a trocar and a hollow cutter cannula through which the needle passes into the bone marrow, then retracting the trocar from the cannula, attaching serum, blood etc. to the cannula and allowing them to reach the bone marrow through the cannula.

In the technique, during intraosseous infusion procedures, generally manual needles are used. Time is one of the most important parameters in the intraosseous infusion procedure. While using manual needles, the needle handle is turned clockwise and counterclockwise by hand and so the needle reaches the bone marrow cavity. Intervention into the marrow channel by using manual needle requires more time than the other systems.

Moreover, spring loaded systems that are activated by pushing the trigger of the spring mechanism and that enable the needle to reach the marrow channel by piercing the bone are also used in bone marrow intervention. While spring loaded systems that are used in intraosseous infusion procedures are the quickest systems in terms of procedure time, some important disadvantages of this system is that only one attempt can be done for intervention and there is the possibility that the powerful spring can cause problems up to breaking the bone.

Today, drive systems are being used in intraosseous infusion procedures. In the driver systems that can perform intervention in a shorter time than the manual needle, there are important disadvantages such as contamination since the driver and the needle are separate and the driver is not sterile and also the charging state of the drive must be constantly monitored.

In the European patent document No.EP2039298B1 of the known state of the art, an apparatus and a method for piercing the bone marrow is described. The apparatus may comprise a manual type body, an input mechanism, a drilling shaft, a gear mechanism, a motor and integrated circuits operated to supply power to the power source and a connector parts that can freely mounted to the circuits.

In the intraosseous infusion procedure performed by this method, first the intraosseous needle is removed from the packaging and installed on the driver. Then, the drive is run and the needle is inserted into the bone of the patient through proximal tibia or proximal humerus regions. When bone marrow cavity is reached, the driver is stopped and removed from the needle. Then, the internal trocar connected to the needle cutting cannula is removed from the needle. The infusion is provided by connecting serum, blood etc. to the cutting cannula. Here, the driver is not sterile. Moreover, the sterility of the needle is compromised when removed from the packaging. In this case, risk of contamination becomes very high.

Moreover, removing the intraosseous needle from the packaging and mounting on the driver takes time. When it is considered that intraosseous infusion procedure is used on very urgent patients (for example with massive bleeding), even 3-5 seconds wasted to install the needle on the drive becomes important.

In addition to waste of time, removing the needle from the packaging and installing on the driver is a very hard task in some cases since the environment is very dark (traffic accidents, military conflict etc.).

Considering the cases known in the art, there is a need to develop a device that is used for intraosseous infusion procedures wherein the internal trocar is integrated with the drive and thus enabling infusion in a both sterile and quick way and additionally where removing the needle from the packaging and installing on the driver is not required.

### Objects of the Invention

The object of the invention is to embody the intraosseous infusion device wherein the internal trocar and the driver are integrated.

Another object of the invention is to embody a device that enables performing intraosseous infusion procedure in a quicker and sterile way by removing from the packaging in a sterile way since the internal trocar and the driver are integrated.

The invention is defined by claim 1. Further embodiments of the invention are defined by the dependent claims. Methods do not form part of the invention.

### Detailed Description of the Invention

The intraosseous infusion device that is embodied to achieve the objects of this invention is shown in the appended figures.

These figures;
**Figure - 1:** Side view of the cannula of the device of the invention.
**Figure - 2:** Perspective view of the cannula of the device of the invention.
**Figure - 3:** The view wherein the cannula is installed on the driver in the device of the invention.
**Figure - 4:** The cross-sectional view of the drive and the internal trocar while the cannula is not attached in the device of the invention.
**Figure - 5:** The side view of the driver and the internal trocar while the cannula is not attached in the device of the invention.
**Figure - 6:** The perspective view of the connection of the cannula and the driver in the device of the invention.
**Figure - 7:** The view of the driver motor circuit.

The parts in the figures are given individual reference numbers and these numbers refer to;
1. Cannula
2. Cutter cannula tip
3. Internal trocar
4. Connection lug
5. Female connection of the drive shaft
6. Shaft connection notch
7. Driver shaft
8. Reductor
9. Motor
10. Battery
11. Driver body
12. Trigger
13. Led light

The intraosseous infusion device of the invention, has the features as claimed in claim 1 and comprises,
- an internal trocar (3) manufactured integral with the driver shaft (7),
- a cannula (1) that is mounted on the driver body (11) which comprises a cutter cannula tip (2) through which the internal trocar (3) and can comprise
- one or more connection lugs (4) that is located on the body of the cannula (1) which enables connection of the cannula (1) with the driver body (11),
- a drive shaft female connection (5) that is connected to the body of the cannula (1),
- one or more shaft connection notches (6) that are located on the driver shaft female connection (5) and that are compatible with the connection lug (4),
- a driver shaft (7) that is directly connected to the driver motor (9) which comprises an internal trocar (3), a driver shaft female connection (5) and a connection notch (6) on the other end,
- a motor (9) connected to the driver shaft (7),
- a reducer (8) that is directly connected to the driver shaft (7) or to the motor (9) by chain/gear/belt mechanisms,
- a led light (13) that is located on the driver body (11) and that illuminates when desired or when the trigger (12) is activated or that dims out when desired or when the trigger (12) is released.

In the device of the invention, the drive driver shaft (7) and the internal trocar (3) are integrated and they are removed from the packaging in a sterile way. In the packaging, the cannula (1) is connected to the driver body (11) and sterilized together. When the infusion procedure is completed, the driver and the internal trocar (3) are removed from the cutter cannula (1) together.

The device of the invention comprises a cutter cannula (1) that enables performing the cutting procedure during the operation.

The device of the invention comprises the internal trocar (3) integrated with the driver shaft (7) which performs the drilling procedure by passing through the cutter cannula (1) and which provides strength to the cutter cannula (1).

The device of the invention can comprise the driver shaft female connection (5) that is connected to the cutter cannula (1) body of the shaft directly connected to the driver motor (9) and that also comprises the internal trocar (3).

The device of the invention can comprise the reductor (8) that enables adjusting the speed of the motor (9) to desired speed and that is connected directly to the driver shaft (7) or to the motor (9) by chain/gear/belt mechanisms.

The device of the invention can comprise the motor (9) that works with direct current and that is used to apply the rotational power to the shaft required for drilling/cutting procedures.

The device of the invention can comprise the battery (10) that provides the electrical current for the motor (9) to operate at appropriate speed and power.

The device of the invention can comprise the closed plastic drive body (11) that keeps all the parts together.

The device of the invention can comprise the trigger (12) that is used to run or stop the motor (9) when desired.

Moreover, the device of the invention can comprise the led light (13) that is located on the drive and that enables performing the infusion procedure in the dark environments.

In said device, the internal trocar (3) and the driver shaft (7) are one-piece. The internal trocar (3) is manufactured integrated with the driver shaft (7). So, the internal trocar (3) is connected directly to the driver shaft (7). There is no connection component between the internal trocar (3) and the driver shaft (7). The driver shaft (7) is directly connected to the cutter cannula (1) by the connection methods known in the art.

When intraosseous infusion procedure is completed, the internal trocar (3) and the driver pull out from the cutter cannula (1) together.

In the device of the invention, since the internal trocar (3) is integrated with the driver in the packaging, the intraosseous infusion procedure is performed very quickly and in a sterile way.

Since the device is sterile, there is no contamination risk originating from the device during procedure.

Since the internal trocar (3) is integral with the driver in the device, the risk of losing the driver or drained charge is eliminated. The invention is defined by the following claims.

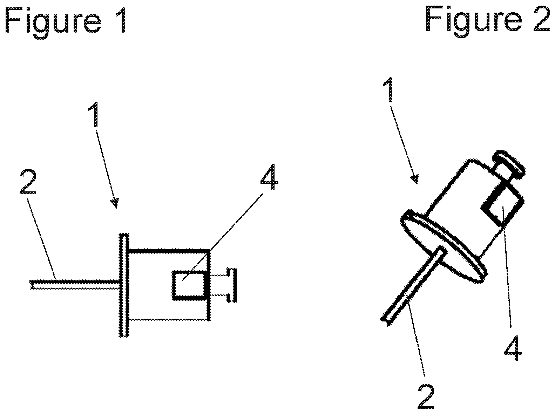

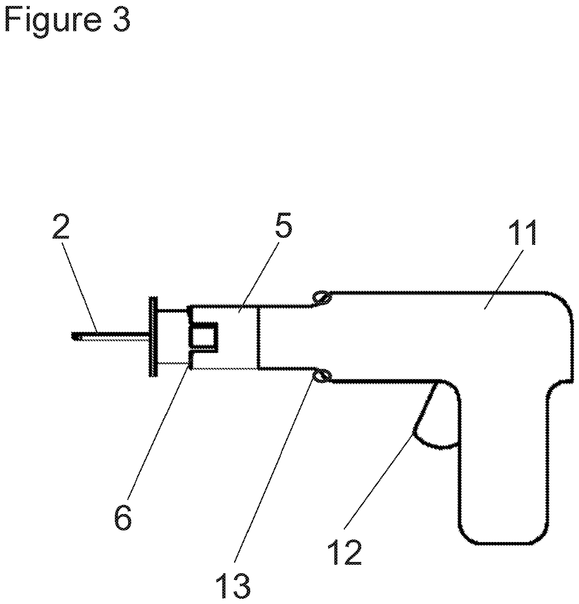

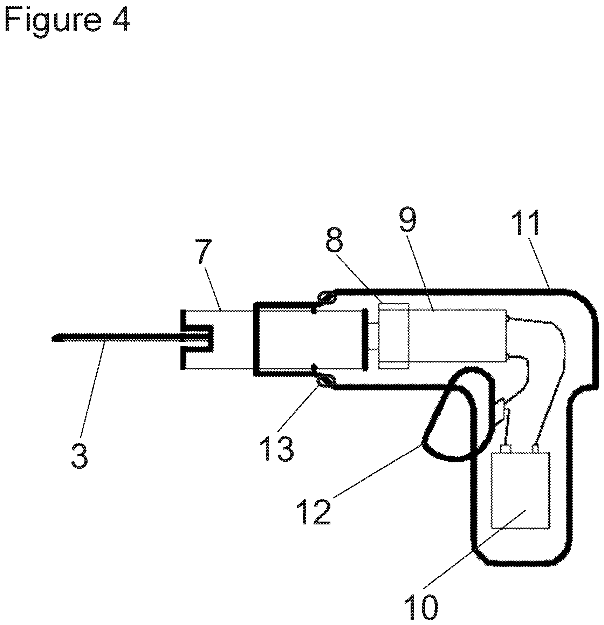

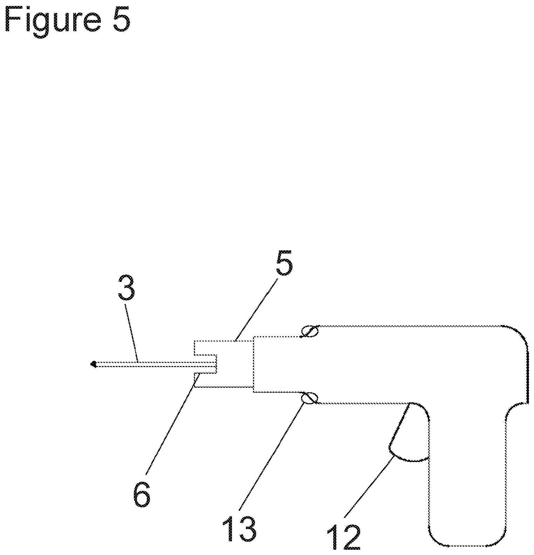

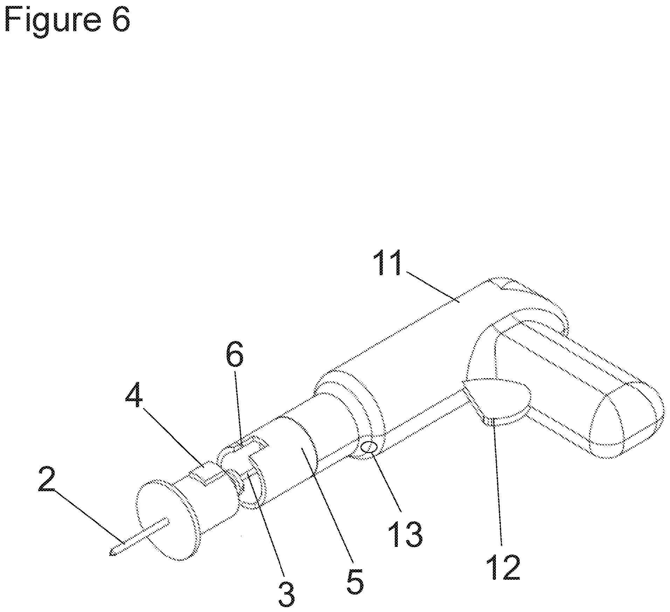

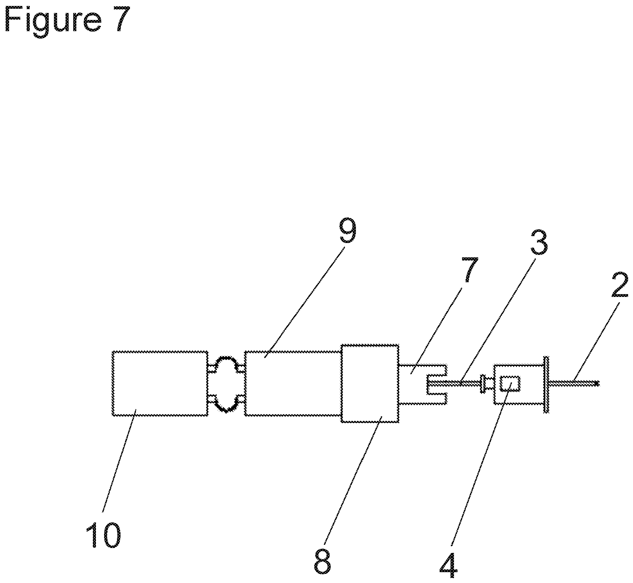

## Claims

1. Intraosseous infusion device comprising,
- a driver body (11) comprising an internal driver motor (9),
- **a cannula (1)** which is installed on the drive body (11), comprising a cutter cannula tip (2) through which an internal trocar (3) passes,
- **a driver shaft (7)** directly connected to the driver motor (9), **characterized in that** said **internal trocar (3)** is manufactured to be integral with the driver shaft (7).

2. Intraosseous infusion device according to Claim 1, **characterized by** comprising one or more connection lugs (4) that is located on the body of a cannula (1) which enables connection of the cannula (1) with the driver body (11).

3. Intraosseous infusion device according to Claim 1, **characterized by** comprising a driver shaft female connection (5) that is connected to the body of the cannula (1).

4. Intraosseous infusion device according to Claim 1, **characterized by** comprising one or more shaft connection notches (6) that is located on the driver shaft female connection (5) and that is compatible with the connection lug (4).

5. Intraosseous infusion device according to Claim 1, **characterized by** comprising a led light (13) that is located on the driver body (11) which illuminates when desired or when the trigger (12) is activated or which dims out when desired or when the trigger (12) is released.

6. Intraosseous infusion device according to Claim 1, **characterized by** comprising a reductor (8) that is directly connected to the driver shaft (7) or to the motor (9) by chain/gear/belt mechanisms.

## Patentansprüche

1. Intraossäre Infusionsvorrichtung umfasst,
- einen Antriebskörper (11) mit einem internen Antriebsmotor (9),
- eine Kanüle (1), die auf dem Antriebskörper (11) installiert ist, mit einer schneidenden Kanülenspitze (2), durch die ein Innentrokar (3) verläuft,
- eine Antriebswelle (7), die direkt mit dem Antriebsmotor (9) verbunden ist, **gekennzeichnet durch** die Tatsache, dass der Innentrokar (3) so hergestellt ist, dass er mit der Antriebswelle (7) einstückig ist.

2. Intraossäre Infusionsvorrichtung nach Anspruch 1, **dadurch gekennzeichnet, dass** sie eine oder mehrere Anschlussnasen (4) umfasst, die sich am Körper einer Kanüle (1) befinden und die Verbindung der Kanüle (1) mit dem Antriebskörper (11) ermöglichen.

3. Intraossäre Infusionsvorrichtung nach Anspruch 1, **dadurch gekennzeichnet, dass** sie einen mit dem Körper der Kanüle (1) verbundenen weiblichen Anschluss (5) für den Antriebsschaft aufweist.

4. Intraossäre Infusionsvorrichtung nach Anspruch 1, **dadurch gekennzeichnet, dass** sie eine oder mehrere Schaftverbindungskerben (6) umfasst, die sich am weiblichen Anschluss (5) des Treiberschafts befinden und die mit der Anschlussnase (4) kompatibel sind.

5. Intraossäre Infusionsvorrichtung nach Anspruch 1, **dadurch gekennzeichnet, dass** sie eine LED-Leuchte (13) umfasst, die sich am Antriebskörper (11) befindet und die auf Wunsch oder bei Betätigung des Auslösers (12) leuchtet oder die auf Wunsch oder beim Loslassen des Auslösers (12) abdimmt.

6. Intraossäre Infusionsvorrichtung nach Anspruch 1, **dadurch gekennzeichnet, dass** sie ein Reduzierstück (8) umfasst, das direkt mit der Antriebswelle (7) oder mit dem Motor (9) durch Ketten-/Getriebe-/Riemenmechanismen verbunden ist.

## Revendications

1. Dispositif de perfusion intra-osseuse comprenant,
- un corps d'entraînement (11) comprenant un moteur d'entraînement interne (9),
- une canule (1) installée sur le corps d'entraînement (11), comprenant une pointe de canule coupante (2) traversée par un trocart interne (3),
- un arbre d'entraînement (7) directement relié au moteur d'entraînement (9), **caractérisé en ce que** ledit trocart interne (3) est fabriqué de manière à être solidaire de l'arbre d'entraînement (7).

2. Dispositif de perfusion intra-osseuse selon la Revendication 1, **caractérisé par le fait qu'**il comprend un ou plusieurs ergots de connexion (4) situés sur le corps d'une canule (1) qui permet la connexion de la canule (1) avec le corps du conducteur (11).

3. Dispositif de perfusion intra-osseuse selon la Revendication 1, **caractérisé par le fait qu'**il comprend un ou plusieurs ergots de connexion (4) situés sur le corps d'une canule (1) qui permet la connexion de la canule (1) avec le corps du conducteur (11).

4. Dispositif de perfusion intra-osseuse selon la Revendication 1, **caractérisé par** la présence d'une ou plusieurs encoches de connexion d'arbre (6) situées sur la connexion femelle de l'arbre d'entraînement (5) et compatibles avec l'ergot de connexion (4).

5. Dispositif de perfusion intra-osseuse selon la Revendication 1, **caractérisé par** la présence d'une lumière LED (13) située sur le corps de l'entraîneur (11) qui s'allume quand on le souhaite ou quand la gâchette (12) est activée ou qui s'éteint quand on le souhaite ou quand la gâchette (12) est relâchée.

6. Dispositif de perfusion intra-osseuse selon la Revendication 1, **caractérisé par** la présence d'un réducteur (8) directement relié à l'arbre d'entraînement (7) ou au moteur (9) par des mécanismes de chaîne, d'engrenage ou de courroie.
